# EUROPÄISCHE PATENTSCHRIFT

(11) **EP 0 623 355 B1**
(45) Veröffentlichungstag und Bekanntmachung des Hinweises auf die Patenterteilung: **28.06.2006**
(21) Anmeldenummer: 94103660.0
(22) Anmeldetag: 09.03.1994
(51) Int. Cl.: A61M 1/00, A61M 25/00, A61M 39/00, A61M 27/00

(54) **Medizinische Gewebe-Drainagevorrichtung**
Medical tissue drainage device
Dispositif médical de drainage de tissus

(30) Priorität: 11.03.1993 DE 4307658
(43) Veröffentlichungstag der Anmeldung: 09.11.1994
(73) Patentinhaber: Mauk, Rudolf, D-22844 Norderstedt (DE); Hahn, Michael Dipl.-Ing., D-22459 Hamburg (DE)
(72) Erfinder: Mauk, Rudolf, D-22844 Norderstedt (DE); Hahn, Michael, Dipl.-Ing., D-22459 Hamburg (DE)
(74) Vertreter: Hansmann, Dierk

(56) Entgegenhaltungen:
- EP-A- 0 091 895
- EP-A- 0 268 733
- WO-A-86/03396
- FR-A- 2 161 950

## Beschreibung

Die Erfindung betrifft eine medizinische Gewebe-Drainagevorrichtung mit einer massiven Nadel, die an ihrem vorderen Ende mit einem das Gewebe durchdringenden Anschliff und an ihrem hinteren Ende mit einem im Gewebe zu plazierenden Drainageschlauch verbunden ist.

Medizinische Gewebe-Drainagevorrichtungen dieser Art werden bei Operationen eingesetzt, um an das Operationsgebiet angrenzendes Gewebe durch Abführen von Blut und anderen Wundsekreten trockenzulegen. Dabei wird die Nadel, die im Regelfall als sogenannte Redon-Nadel bezeichnet wird, durch das trockenzulegende Gewebe hindurchgestoßen und der am hinteren Ende der Nadel befestigte Drainageschlauch nachgezogen, bis er mit einem perforierten Bereich das Gewebe an der vorgesehenen Stelle durchsetzt. Anschließend wird der aus einem weichen Kunststoffmaterial bestehende Drainageschlauch von der Nadel getrennt und die Nadel entfernt. Die Trennung der Nadel vom Drainageschlauch erfolgt im Regelfall durch Abschneiden des Drainageschlauches. Es ist jedoch auch möglich, die Nadel aus dem Drainageschlauch herauszudrehen.

Aus der DE-A-2 335 560 ist bereits eine derartige Drainagevorrichtung mit einer Nadel bekannt, die an ihrem vorderen Ende eine in eine Spitze auslaufende Messerschneide und an ihrem hinteren Ende einen mit umlaufenden Sägezahn-Rillen versehenen Zapfen aufweist, auf den das vordere Ende des Drainageschlauches unter Aufweitung aufgezogen ist, wobei sich die Rillen von innen her in die Wand des Drainageschlauches einpressen und verhindern, daß sich der Drainageschlauch von der Nadel lösen kann. Weiter sind auch Nadeln bekannt, bei denen der Zapfen an Stelle der umlaufenden Rillen ein Außengewinde aufweist.

Die Nadeln dieser Drainagevorrichtungen werden häufig nur einmal verwendet, da das Entfernen des nach dem Durchschneiden an der Nadel verbleibenden Schlauchstückes, die Reinigung des Außengewindes bzw. der Rillen von durch den Drainageschlauch eingedrungenen Wundsekreten, das Sterilisieren der Nadel und das anschließende Anbringen eines neuen sterilen Drainageschlauches zu aufwendig sind, insbesondere auch deshalb, weil zum Anbringen des neuen Drainageschlauches dessen vorderes Ende unter sterilen Bedingungen unter gleichzeitiger Aufweitung auf den Zapfen geschoben werden müßte.

Eine mehrmalige Verwendung der Nadeln der eingangs genannten Gewebe-Drainagevorrichtungen wird weiter dadurch erschwert, daß die Nadeln vor einer erneuten Verwendung nachgeschliffen werden müssen, um sicherzustellen, daß ihre Spitze noch ausreichend scharf ist. Bei der bekannten Nadel wird das Anschleifen dadurch verhältnismäßig teuer, daß relativ große Flächen in einem vorgegebenen Winkel zueinander abgeschliffen werden müssen. Dazu muß eine vergleichsweise aufwendige Schleifvorrichtung eingesetzt werden.

Ein weiteres Problem besteht darin, daß bei der Verwendung der in Längsrichtung angeschliffenen Nadel das Gewebe relativ weit aufgetrennt wird, ohne daß im Blick auf die Verlegung der Wunddrainage dazu eine Notwenigkeit besteht. Diese unbeabsichtigte Weiterauftrennung geschieht bei richtiger Verwendung der Nadel in erster Linie dadurch, daß die in Längsrichtung der Nadel verlaufende Schneidkante das Gewebe aufschneidet. Wird durch dieses aufgeschnittene Gewebe die Nadel mit ihrem Halteteil und dem Drainageschlauch hindurchgezogen, so besteht die große Gefahr, daß das Gewebe unbeabsichtigt im Bereich der Schnittwunde weiterreißt, wenn es aufgeweitet wird.

Nach der EP-A-0091895 ist eine Vorrichtung zur Entwässerung einer Harnblase bekannt, wobei eine Nadel und ein Katheter angeordnet sind und die Nadel über eine Führungssonde durch die Harnröhre in die Harnblase eingeführt wird. Die Nadel ist dabei mit dem vorgenannten Mangel direkt mit dem Schlauch verbunden. Die Verbindung erfolgt durch einen Ansatz am Nadelende mit einem Gewinde, das in die Öffnung des Schlauches mit einem Sackloch mit Gewinde gedreht wird. Alternativ wird ein gewindefreier Ansatz der Nadel in den Schlauch gesteckt und mit diesem verklebt. Die Erstellung eines Innengewindes in einem Sackloch am Schlauchende ist problematisch und aufwendig durchführbar. Das Eindrehen der Nadel in einen flexiblen Schlauch ohne Gewinde ist mühsam und dehnt den Schlauch auf. Hieraus ergeben sich Durchmesserdifferenzen, die ein Durchführen der Nadel erschweren. Auch das Entfernen der Nadel vom Schlauch für eine Mehrfachbenutzung der Nadel ist aufwendig.

Aufgabe der Erfindung ist es, eine medizinische Gewebe-Drainagevorrichtung der eingangs genannten Art so zu verbessern, daß bei ihrer Verwendung das Gewebe weitgehend geschont und eine direkte Verbindung ohne Querschnittsveränderung zwischen Nadel und Schlauch als Kupplung ermöglicht wird.

Diese Aufgabe wird erfindungsgemäß zum einen dadurch gelöst, daß die Nadel einen Anschliff als Kanülenanschliff aufweist und zwischen der Nadel und dem Drainageschlauch ein separates Verbindungselement angeordnet ist, wobei das Verbindungselement einerseits lösbar mit der Nadel über eine Schraubverbindung und das Verbindungsstück andererseits unlösbar bzw. schwer lösbar in einen Drainageschlauch einsetzbar ist sowie die Schraubverbindung durch einen axial überstehenden, mit einem Außengewinde versehenen Zapfen in eine mit einem korrespondierenden Innengewinde versehene axiale Ausnehmung des Verbindungselementes bzw. des hinteren Endes der Nadel einschraubbar ist.

Bei dieser Gewebe-Drainagevorrichtung besteht ein wesentlicher Vorteil darin, daß der Drainageschlauch mit der Nadel sehr präzise verbunden werden kann, so daß eine einfache Verlegung des Drainageschlauches möglich ist. Dabei ist das Verbindungselement so genau gefertigt, daß es den Drainageschlauch aufnehmen kann, ohne daß dieser nach seiner Anbringung am Verbindungselement über den Querschnitt der Nadel herausragt. Beim Einziehen des Drainageschlauches wird daher ein Aufreißen des Gewebes zuverlässig verhindert.

Darüber hinaus wird auch das Gewebe durch die äußere Begrenzung des Anschliffes weitgehend geschont. Beim Eindringen des Anschliffes in das Gewebe wird dieses gleichmäßig aufgeweitet, ohne daß es an einer bestimmten Stelle aufreißt. Unabhängig davon, ob die Nadel falsch oder richtig geführt wird, entsteht im Gewebe eine Öffnung, die für die Aufnahme des Drainageschlauches optimal geeignet ist.

Eine Verbindung zwischen dem Verbindungsstück und dem Schlauch wird bereits gewerbsmäßig vor der Verwendung der Drainagevorrichtung durch den Hersteller vorgenommen. Diese Verbindung ist für den vorgesehenen Zweck optimal geeignet, da der Schlauch bündig mit der Außenoberfläche des Verbindungsstückes abschließt und über diesen nicht hinausragt. Vor dem Einsatz der Drainagevorrichtung kann die Nadel leicht mit dem Verbindungsstück verbunden werden, ohne daß die Gefahr einer nichtsterilen Behandlungsweise besteht. Durch die Anpassung des Verbindungsstückes an den Querschnitt der Nadel entsteht auch in diesem Bereich keine störende Kante, die beim Verlegen des Drainageschlauches das Gewebe ungewollt zerstören könnte.

Darüber hinaus kann nach der Verwendung der Nadel diese auf einfache und schnelle Weise, durch Abschrauben, von dem Verbindungsstück getrennt werden, so daß sie im Anschluß daran gesäubert, sterilisiert und nochmals verwendet werden kann. Durch Ankopplung eines neuen sterilen Drainageschlauches kann diese unter Verwendung der bereits benutzten, inzwischen aber gesäuberten und sterilisierten Nadel zum Zwecke der Drainage verlegt werden.

Um zu gewährleisten, daß das Gewinde der Nadel nicht mit Wundsekreten in Berührung kommt, die eine Reinigung und Sterilisation erschweren, sieht eine weitere bevorzugte Ausgestaltung der Erfindung vor, daß das Verbindungselement mit einer die Ausnehmung bzw. den Zapfen ringförmig umgebenden Dichtfläche an einer ihr gegenüberliegenden Dichtfläche der Nadel anliegt. Dabei ist das Verbindungselement hinter der Ausnehmung bzw. dem Zapfen außerdem vorzugsweise massiv ausgebildet, so daß vom Drainageschlauch aus kein Wundsekret durch das Verbindungselement zum Gewinde hindurchtreten kann.

Wird die Gewindeverbindung gemäß einer vorteilhaften Ausgestaltung der Erfindung weiter so ausgebildet, daß das Außengewinde auf dem über das hintere Ende der Nadel überstehenden Zapfen einen gegenüber dem Innendurchmesser des Drainageschlauches etwas größeren Innendurchmesser aufweist, so kann ein abgeschnittener Drainageschlauch im Notfall auch ohne ein Verbindungselement mit der Nadel verbunden werden.

Im folgenden wird die Erfindung anhand mehrerer in der Zeichnung in schematischer Weise dargestellter Ausführungsbeispiele näher erläutert. Es zeigen:
Fig. 1: eine Seitenansicht einer erfindungsgemäßen Gewebe-Drainagevorrichtung mit einem zwischen der Nadel und dem Drainageschlauch angeordneten Verbindungselement.
Fig. 2 : eine teilweise geschnittene Seitenansicht einer Ausführungsform eines Verbindungselementes.
Fig. 3 : eine vergrößerte Seitenansicht des vorderen Endes der Nadel aus Fig. 1,

Die in der Zeichnung dargestellte Drainagevorrichtung zum Trokkenlegen von Gewebe besteht im wesentlichen aus einer Nadel (2) und einem Drainageschlauch (4), der mittels eines zwischen der Nadel (2) und dem Drainageschlauch (4) angeordneten Verbindungselementes (6) am hinteren Ende (8) der Nadel (2) lösbar befestigbar ist. Der aus einem weichen transparenten Kunststoffmaterial, beispielsweise aus Polyethylen bestehende Drainageschlauch (4) weist an seinem hinteren Ende (9) einen mit Perforationen (10) versehenen Bereich (12) auf. Sein vorderes Ende (13) ist fest mit dem Verbindungselement (6) verbunden.

Die Nadel (2) besteht im wesentlichen aus einem Halteteil (14) und einem vorderen Ende (16), dessen Längsachse (18) mit der Längsachse (20) des Halteteiles (14) einen stumpfen Winkel einschließt. Das Halteteil (14) und der an das Halteteil (14) anschließende Teil des vorderen Endes (16) weisen einen kreisförmigen Querschnitt auf, dessen Außendurchmesser dem Außendurchmesser des im Querschnitt ringförmigen Drainageschlauches (4) entspricht. Das vordere Ende (16) besitzt einen dem Halteteil (14) abgewandten Einstichteil (89).

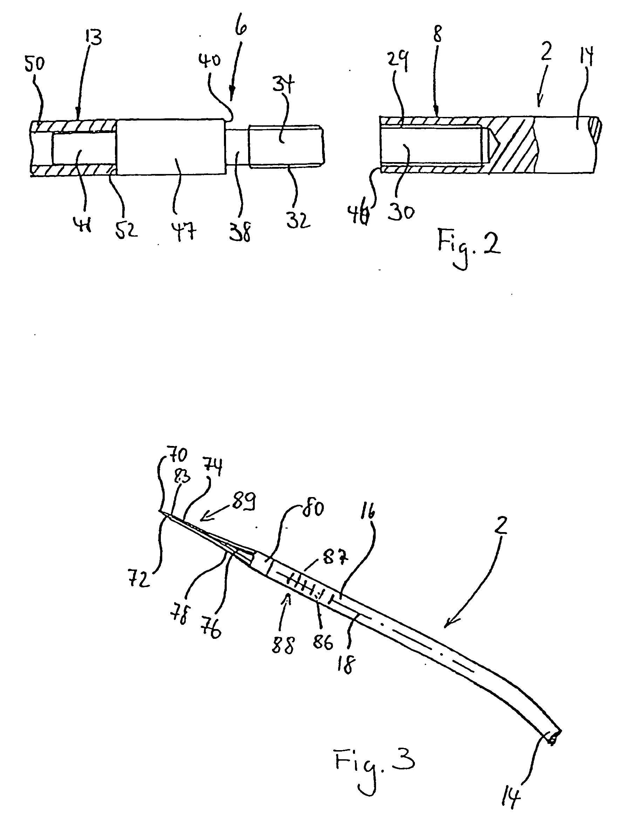

Die lösbare Befestigung des Verbindungselementes (6) an der Nadel (2) erfolgt mittels einer Schraubverbindung, die gemäß Figur 2 eine in axialer Richtung in das hintere Ende (8) des Halteteiles (14) eingebrachte, mit einem Innengewinde (29) versehene Bohrung (30) aufweist in die ein mit einem Außengewinde (32) versehener, axial nach vorne über das Verbindungselement (6) überstehender Zapfen (34) einschraubbar ist. Das Außengewinde (32) und das Innengewinde (29) können jeweils außer als eingängiges Gewinde auch als zwei- oder dreigängiges Gewinde ausgebildet sein. In der Ausführungsform als zwei- oder dreigängiges Gewinde kann das Verbindungselement (6) mit wenigen Drehungen an die Nadel (2) angekoppelt oder von dieser gelöst werden.

An seinem vorderen Stirnende weist das Verbindungselement (6) den Fuß (38) des Zapfens (34) ringförmig umgebende radiale Dichtfläche (40) auf, die beim Zusammenschrauben von Verbindungselement (6) und Nadel (2) gegen eine entsprechende Ringfläche (44) bzw. (46) am hinteren Ende (8) des Halteteiles (14) angepreßt wird und verhindert, daß Blut oder andere Wundsekrete während des Einbringens der Drainagevorrichtung in die Schraubverbindung eindringen. Die Abdichtung kann dadurch verbessert werden, daß der Fuß (38) des Zapfens (22, 34) einen Durchmesser aufweist, der geringfügig kleiner als der Nenndurchmesser des Außengewindes (24, 32) ist.

Um zu gewährleisten, daß sich nach dem Durchschneiden des Drainageschlauches (4) die Nadel (2) stets im Bereich der Schraubverbindung vom Verbindungselement (6) löst, ist eine Verbindungsstelle (51) zwischen Drainageschlauch (4) und Verbindungselement (6) so ausgebildet, daß sie nicht oder nur schwer lösbar ist. Dies kann beispielsweise dadurch erfolgen, daß das Verbindungselement (6), einen in axialer Richtung nach hinten überstehenden Zapfen (48) mit kreisförmigem Querschnitt aufweist, dessen Außendurchmesser dem Innendurchmesser des Drainageschlauches (4) entspricht, wobei das vordere Ende des Drainageschlauches (4) nach Auftragen eines Klebstoffes auf den Zapfen (48) bis zum Anschlagen gegen eine den Fuß des Zapfens (48) umgebende Ringfläche (52) auf diesen aufgeschoben wird. Dabei ist die Verbindungsstelle (51) so ausgebildet, daß der Drainageschlauch (4) mit seiner äußeren Wandung (53) über eine das Verbindungselement (6) begrenzende Außenwandung (7) nicht hinausragt. Auf diese Weise wird zuverlässig vermieden, daß beim Durchstechen des Gewebes mit Hilfe der Nadel (2) im Bereich der Verbindungsstelle (51) das Gewebe von einem über die Außenwandung (7) überstehenden Teil des Drainageschlauches (4) aufgerissen wird.

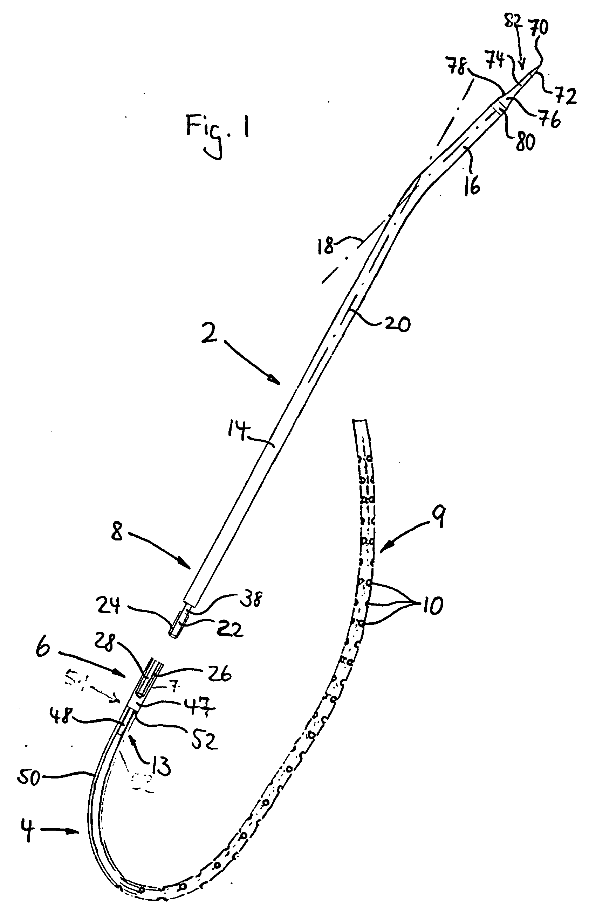

Das vordere Ende der Nadel (2) besitzt einen Anschliff (82), dessen äußere Begrenzung das Gewebe gleichmäßig aufweitet und der frei von einer in Längsrichtung des vorderen Endes (16) verlaufenden Längskante ausgebildet ist. Dieser Anschliff (82) wird von Umfangsflächen begrenzt, die rotationssymmentrisch zur Längsachse (18) ausgebildet sind. Bei dem in Figur 1 und 3 dargestelltem Ausführungsbeispiel, das eine Nadel mit sehr kleinem Querschnitt zeigt, schließt sich dabei an die punktförmige Spitze (70) der Nadel (2) nach hinten zu eine im wesentlichen kegelförmige Schliffläche (72) an, deren Mantellinie mit der Längsachse (18) einen Winkel von etwa 25 Grad einschließt. Diese kegelförmige Schliffläche (72) erweitert sich bis zu einer Grundfläche (83), deren Durchmesser etwa halb so groß wie der Durchmessers des Halteteils (14) ist. Im Anschluß an die Grundfläche (83) schließt sich in Richtung auf das hintere Ende (8) ein zylindrischer Abschnitt (74) an, der gegenüber der Grundflläche (83) verjüngt ist.

Die Verjüngung des zylindrischen Abschnittes (74) gegenüber der Grundfläche (83) ist etwa 2/3 vom Durchmesser der Grundfläche. Vom zylindrischen Abschnitt (74) zur Grundfläche (83) steigt die Oberfläche in einem sanften Anstieg an. Der zylindrische Abschnitt (74) ist etwa dreimal so lang wie die Höhe des Kegels, der von der kegelförmigen Schnittfläche (72) gebildet wird.

Zwischem dem zylindrischen Abschnitt (74) und dem Halteteil (14) ist ein sanfter Übergang (76) angeordnet. Dieser sanfte Übergang (76) wird von einer Fläche (78) begrenzt, die im Profil konkav gekrümmt ist. Sie ist etwa doppelt so lang wie der zylindrische Abschnitt (74). Dieser sanfte Übergang (76) mündet über einen konischen Nadelabschnitt (80) in den Halteteil (14) ein. Der konische Nadelabschnitt (80) besitzt etwa 1/3 der Länge des Überganges. (76). Der konische Nadelabschnitt (80) besitzt eine Mantellinie, mit der die Längsachse (18) des vorderen Endes (16) einen Winkel von etwa 10 Grad einschließt.

## Patentansprüche

1. Medizinische Gewebe-Drainagevorrichtung mit einer massiven Nadel (2), die an ihrem vorderen Ende mit einem das Gewebe durchdringenden Anschliff (82) und an ihrem hinteren Ende (8) mit einem im Gewebe zu plazierenden Drainageschlauch (4) verbunden ist, wobei die Nadel (2) einen Anschliff (82) als Kanülenanschliff (84) aufweist **dadurch gekennkzeichnet, daß** zwischen der Nadel (2) und dem Drainageschlauch ein separates verbindungselement (6) angeordnet ist, wobei das Verbindungselement (6) einerseits lösbar mit der Nadel (2) über eine Schraubverbindung und das Verbindungsstück (6) andererseits unlösbar bzw. schwer lösbar in einen Drainageschlauch (4) einsetzbar ist sowie die Schraubverbindung durch einen axial überstehenden, mit einem Außengewinde (32) versehenen Zapfen (34) in eine mit einem korrespondierenden Innengewinde (29) versehene axiale Ausnehmung (28) des verbindungselementes (6) bzw. des hinteren Endes (8) der Nadel (2) einschraubbar ist.

2. Gewebe-Drainagevorrichtung nach Anspruch 1, **dadurch gekennzeichnet, daß** das verbindungselement (6) mit einer die Ausnehmung (28) bzw. den Zapfen (34) ringförmig umgebenden Dichtfläche (40) gegen eine gegenüberliegende Dichtfläche (44, 46) der Nadel (2) anpreßbar ist.

3. Gewebe-Drainagevorrichtung nach einem der Ansprüche 1 oder 2, **dadurch gekennzeichnet, daß** der Zapfen (22, 34) einen Fuß (38) mit einem gegenüber dem Innendurchmesser des Außengewindes (24, 32) geringeren Außendurchmesser aufweist.

4. Gewebe-Drainagevorrichtung nach einem der Ansprüche 1 bis 3, **dadurch gekennzeichnet, daß** zwischen dem Verbindungselement(6) und dem Drainageschlauch (4) eine Verbindungssstelle (51) vorgesehen ist, in deren Bereich das Verbindungselement (6) eine Außenwandung (7) aufweist, die mindestens einen gleichgroßen Außendurchmesser aufweist wie eine den Drainageschlauch (4) begrenzende äußere wandung (53).

## Claims

1. Medical tissue drainage device with a solid needle (2), which is provided with a cutting tip (82) penetrating the tissue at its front end and is connected at its rear end (8) to a drainage tube (4) to be positioned in the tissue, and the needle (2) has a cutting tip (82) as a cannula cutter (84), **characterised in that** a separate connecting element (6) is disposed between the needle (2) and the drainage tube, which connecting element (6) can be releasably connected to the needle (2) by means of a screw connection at one end, and the connecting piece (6) can be inserted in a drainage tube (4) so that it can not be detached or is difficult to detach at the other end, and the screw connection can be screwed by means of an axially protruding pin (34) provided with an external thread (32) into a matching internal thread (29) of an axial recess (28) of the connecting element (6) or rear end (8) of the needle (2).

2. Tissue drainage device as claimed in claim 1, **characterised in that** the connecting element (6) can be pressed by means of a seal surface (40) surrounding the recess (28) or pin (34) in an annular shape against an oppositely lying seal surface (44, 46) of the needle (2).

3. Tissue drainage device as claimed in one of claims 1 or 2, **characterised in that** the pin (22, 34) has a base (38) with an external diameter smaller than the internal diameter of the external thread (24, 32).

4. Tissue drainage device as claimed in one of claims 1 to 3, **characterised in that** a connection point (51) is provided between the connecting element (6) and the drainage tube (4), in the region of which the connecting element (6) has an external wall (7) with an external diameter at least equal in size to an external wall (53) bounding the drainage tube (4).

## Revendications

1. Dispositif médical de drainage de tissus avec une aiguille massive (2) qui est reliée à son extrémité antérieure à un affûtage (82) pénétrant dans le tissu et à son extrémité postérieure (8) à un tuyau flexible de drainage (4) à placer dans le tissu, l'aiguille (2) présentant un affûtage (82) comme affûtage de canule (84), **caractérisé en ce qu'**un élément de connexion (6) séparé est disposé entre l'aiguille (2) et le tuyau flexible de drainage, l'élément de connexion (6) pouvant être fixé, d'une part, de manière amovible à l'aiguille (2) par l'intermédiaire d'une connexion filetée et l'élément de connexion (6) pouvant être inséré, d'autre part, de manière inamovible ou difficilement amovible dans un tuyau flexible de drainage (4) et la connexion filetée pouvant être vissée, au moyen d'un tourillon (34) dépassant axialement, pourvu d'un filetage extérieur (32), dans un évidement axial (28) de l'élément de connexion (6) ou encore de l'extrémité arrière (8) de l'aiguille (2), lequel évidement axial est pourvu d'un filetage intérieur (29) correspondant.

2. Dispositif médical de drainage de tissus selon la revendication 1, **caractérisé en ce que** l'élément de connexion (6) peut être pressé avec une surface étanche (40) entourant de manière annulaire l'évidement (28) ou encore le tourillon (34) contre une surface étanche (44, 46) opposée de l'aiguille.

3. Dispositif médical de drainage de tissus selon une des revendications 1 ou 2, **caractérisé en ce que** le tourillon (22, 34) présente un pied (38) avec un diamètre extérieur plus petit que le diamètre intérieur du filetage extérieur (24, 32).

4. Dispositif médical de drainage de tissus selon une des revendications 1 à 3, **caractérisé en ce qu'**il est prévu entre l'élément de connexion (6) et le tuyau flexible de drainage (4) une jonction (51) dans la région de laquelle l'élément de connexion (6) présente une paroi extérieure (7) qui présente au moins un diamètre extérieur de même grandeur qu'une paroi extérieure (53) limitant le tuyau de flexible de drainage (4).
